# FASCICULE DE BREVET EUROPEEN

(11) **EP 1 268 374 B1**
(45) Date de publication et mention de la délivrance du brevet: **05.11.2003**
(21) Numéro de dépôt: 01921431.1
(22) Date de dépôt: 27.03.2001
(51) Int. Cl.: C07C 13/48, C07C 2/86, C09K 5/10

(54) **COMPOSITIONS DE MONO- ET POLYBENZYL-1,2,3,4-TETRAHYDRONAPHTALENES, UTILISATION DESDITES COMPOSITIONS OU D'UN MELANGE D'ISOMERES DU MONOBENZYL-1,2,3,4-TETRAHYDRONAPHTALENE COMME FLUIDE DE TRANSFERT DE CHALEUR**
ZUSAMMENSETZUNGEN VON MONO- UND POLYBENZYL-1,2,3,4-TETRAHYDRONAPHTHALEN, VERWENDUNG DIESER ZUSAMMENSETZUNGEN ODER VON EINER ISOMERENMISCHUNG VON MONOBENZYL-1,2,3,4-TETRAHYDRONAPHTHALEN ALS WÄRMEÜBERTRAGUNGSMITTEL
MONO- AND POLYBENZYL-1,2,3,4-TETRAHYDRONAPHTHALENE COMPOSITIONS, USE OF SAID COMPOSITIONS OR MIXTURE OF MONOBENZYL-1,2,3,4-TETRAHYDRONAPHTHALENE ISOMERS AS HEAT TRANSFER FLUID

(30) Priorité: 29.03.2000 FR 0003946; 01.02.2001 FR 0101355
(43) Date de publication de la demande: 02.01.2003
(73) Titulaire: Atofina, 92800 Puteaux (FR)
(72) Inventeur: COMMANDEUR, Raymond, F-38220 Vizille (FR)
(86) Numéro de dépôt international: FR0100932
(87) Numéro de publication internationale: WO01072672

(56) Documents cités:
- DATABASE WPI Section Ch, Week 197521 Derwent Publications Ltd., London, GB; Class E14, AN 1975-34982W XP002152852 & JP 49 105781 A (MARUZEN PETROLEUM OIL), 7 octobre 1974 (1974-10-07) cité dans la demande

## Description

La présente invention concerne des compositions de mono- et polybenzyl-1,2,3,4-tetrahydronaphtalènes.

Ces compositions comprennent un mélange de composés (Y), (Y1) et (Y2) suivants :
◆ (Y) est un mélange d'isomères du monobenzyl-1,2,3,4-tetrahydronaphtalène de formule : 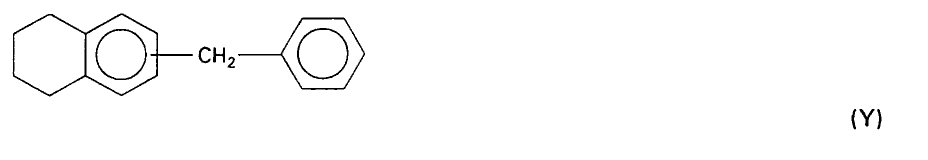
◆ (Y1) est un mélange de composés de monobenzylation de (Y) de formules : 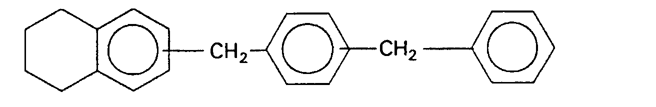 et 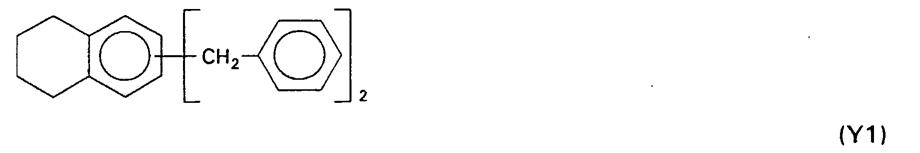
◆ (Y2) est un mélange de composés de mono- ou polybenzylation de (Y1) de formules : 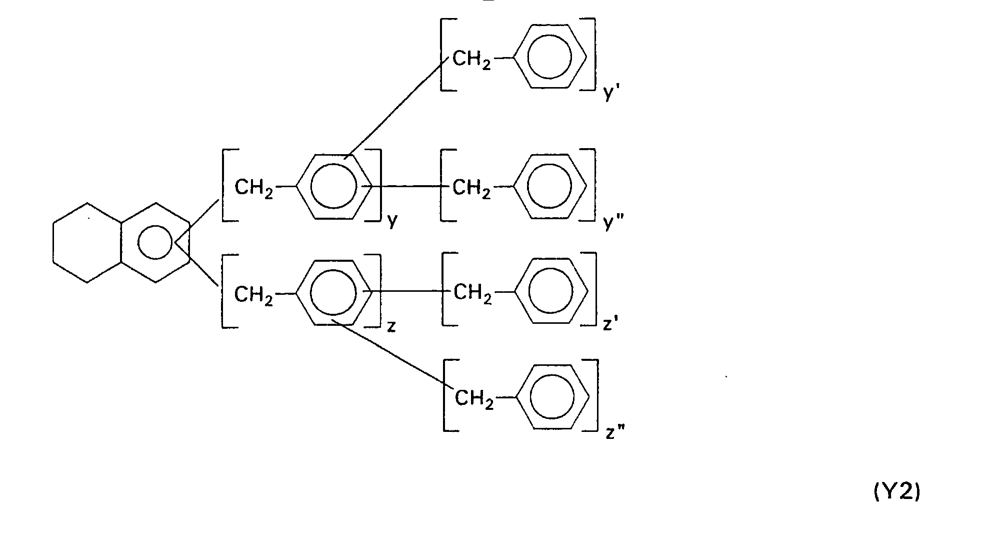 avec y et z=0, 1 ou 2 ; y', y", z', z"=O ou 1 sachant que y+z n'est jamais égal à 0, que y'+y"+z'+z"≥1 et que y+z+y'+y"+z'+z" ≥3.

L'invention concerne également l'utilisation desdites compositions ou bien d'un mélange d'isomères du monobenzyl-1,2,3,4-tétrahydronaphtalène de formule : 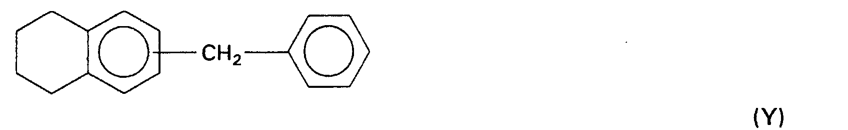 comme fluide de transfert de chaleur ou caloporteur.

Les caloporteurs sont largement utilisés pour assurer un contrôle rigoureux des températures dans les opérations des industries chimiques et doivent posséder un certain nombre de caractéristiques physicochimiques.

Ainsi, les fluides de transfert de chaleur qui doivent être utilisés dans une plage très large de températures, c'est-à-dire allant de -30°C à +400°C, doivent avoir, outre un bon transfert de chaleur, une température d'ébullition à pression atmosphérique élevée, une bonne stabilité thermique, une viscosité faible sur une grande plage de températures, même à basse température lors, notamment de leur mise en oeuvre, une faible tendance à corroder les matériaux de l'appareillage, une faible sensibilité à l'oxydation. Ils doivent également entraîner de faibles risques pour l'environnement en cas de fuites et également de faibles risques d'incendie.

Parmi tous ces critères, la stabilité thermique est un critère déterminant et est le souci des fabricants et producteurs de caloporteurs.

Typiquement, lorsqu'un fluide de transfert de chaleur se dégrade, il se forme à la fois des produits volatils qui abaissent le point d'éclair du fluide de transfert de chaleur et des produits lourds qui augmentent la viscosité et abaissent ainsi le coefficient de transfert de chaleur.

De nombreuses publications proposent des produits tendant à répondre à l'ensemble des critères précédemment mentionnés mais la gamme de produits utilisables notamment à pression atmosphérique dans une gamme de température allant de l'ambiante à environ 350°C est limitée.

On pourra trouver dans l'article de COMMANDEUR et al. intitulé : "Une nouvelle famille de fluides thermiques hautes performances (Inf. Chimie n° 376, 1996 pages 93-96) et dans KIRK-OTHMER Encyclopédia of Chemical technologie - 4^{ème} édition vol. 12 pages 993 à 1006 une liste des principaux fluides de transfert de chaleur commercialement disponibles.

A titre d'illustration de tels produits, on citera notamment les mélanges d'isomères du dibenzyltoluène, les terphényles partiellement hydrogénés, les alkylats du benzène, les mélanges de biphényle et d'oxyde de diphényle.

Furukawa Y. et col. décrivent, dans la demande de brevet JP 74 105 781 publiée le 7 octobre 1974, des fluides de transfert de chaleur constitués essentiellement par des composés de formule : 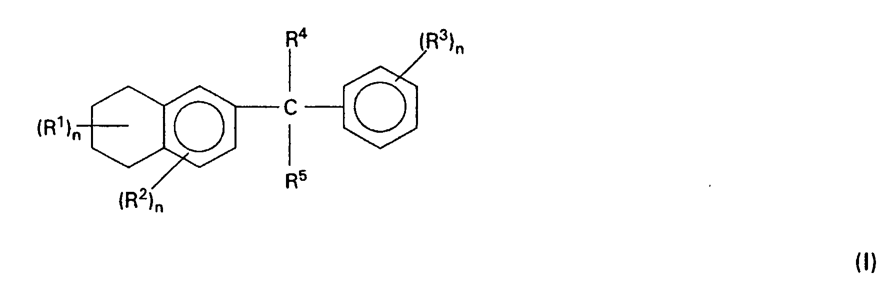 dans laquelle R¹, R², R³, R⁴ et R⁵ est choisi parmi l'atome d'hydrogène et un radical alkyle bas tel que CH₃- et n est égal à 1 ou 2.

La stabilité thermique de ces produits a été évaluée sous pression d'azote à des températures au plus égales à 340°C.

Ainsi, le 1-phényl-1-(5,6,7,8-tétrahydro-2-naphtyl)éthane (formule (I) dans laquelle R¹ = R² = R³ = R⁴ = H, R⁵ = CH₃, n = 1) a été testé à 340°C sous une pression d'azote de 1,47 × 10² kPa (15 kg/cm²) pendant 14 jours et ne montre aucun changement notable du point d'éclair, de sa viscosité et de sa couleur.

Les compositions de mono- et polybenzyl-1,2,3,4-tétrahydronaphtalène ainsi que le mélange d'isomères du monobenzyl-1,2,3,4-tetrahydronaphtalène de la présente invention répond globalement aux critères précédemment mentionnés. Notamment, ils présentent une stabilité thermique qui leur permettent d'être utilisés à des températures aussi élevées que 370°C à des pressions égales ou supérieures à la pression atmosphérique tout en conservant leurs excellentes propriétés de transfert de chaleur.

Selon la présente invention, les composés (Y), (Y1) et (Y2) se trouvent être dans les compositions selon des proportions pondérales suivantes :
- composés de formule (Y) compris entre 60 % et 90 %,
- composés de formule (Y1) compris entre 9 % et 35 %,
- composés de formule (Y2) compris entre 0,1 % et 10 %.

Selon la présente invention, le mélange d'isomères de monobenzyl-1,2,3,4-tétrahydronaphtalène comprend un mélange de 30 % à 40 % en poids de 5-benzyl-1,2,3,4-tétrahydronaphtalène et de 70 % à 60 % en poids de 6-benzyl-1,2,3,4-tétrahydronaphtalène.

Les compositions de mono- et polybenzyl-1,2,3,4-tétrahydronaphtalène ainsi que le mélange d'isomères du monobenzyl-1,2,3,4-tétrahydronaphtalène peuvent être obtenues en faisant réagir le chlorure de benzyl sur un large excès molaire de 1,2,3,4-tétrahydronaphtalène en présence d'un halogénure minéral ou encore d'un acide protonique.

On utilisera un rapport molaire 1,2,3,4-tétrahydronaphtalène / chlorure de benzyl de préférence égal à environ 4 lorsque l'on souhaite obtenir les compositions comprenant un mélange de composés (Y), (Y1) et (Y2) et, au moins égal à 4 et, de préférence compris entre 8 et 12 lorsque l'on souhaite obtenir le mélange d'isomère du monobenzyl-1,2,3,4-tétrahydronaphtalène.

Cette réaction a lieu en pratique à une température comprise entre 30°C et 150°C et, de préférence entre 50°C et 100°C.

Parmi les halogénures minéraux, on peut utiliser le chlorure ferrique, le trichlorure d'antimoine, le tétrachlorure de titane ou encore le chlorure d'aluminium à des teneurs pondérales par rapport aux réactifs mis en oeuvre compris habituellement entre 50 ppm et 1 % et de préférence entre 100 ppm et 0,5 %. De préférence, on utilisera le chlorure ferrique. Les acides protoniques peuvent également être utilisés : l'acide sulfurique par exemple à une concentration pondérale comprise entre 70 et 95 %. Il est aussi possible d'employer les zéolithes ou encore certains oxydes minéraux.

Après distillation du 1,2,3,4-tétrahydronaphtalène en excès, on procède à l'élimination de l'halogénure minérale ou de l'acide protonique par toute technique connue telle que : lavage à l'eau puis séchage dans le cas où l'on utilise un acide protonique, traitement au méthylate de sodium tel que décrit dans le brevet EP 306 398 B1 dans le cas où l'on utilise un halogénure minéral.

Le produit ainsi traité est soumis, soit à une évaporation flash pour éliminer les traces d'impuretés provenant soit des matières premières ou du procédé, ou ayant une origine accidentelle ainsi que les éventuels résidus catalytiques ; soit à une distillation fractionnée de façon à obtenir des fractions comprenant les composés (Y), (Y1) et/ou (Y2). A partir de ces fractions, on peut préparer des compositions ayant des teneurs en composés (Y), (Y1) et (Y2) bien définies.

La caractérisation des composés (Y), (Y1) et (Y2) et leurs teneurs dans les diverses fractions de distillation ainsi que la caractérisation du mélange d'isomères du monobenzyl-1,2,3,4-tétrahydronaphtalène peuvent être réalisées par analyse RMN du protonet du carbone 13 ainsi que par le couplage chromatographie en phase gazeuse / spectrométrie de masse.

On ne sortirait pas du cadre de l'invention si, pour la préparation des compositions comprenant les composés (Y), (Y1) et (Y2) on utilisait, à la place du 1,2,3,4-tétrahydronaphtalène, un mélange de composés plus ou moins hydrogénés du naphtalène. Ces mélanges comprennent généralement de 80 % 90 % en poids du 1,2,3,4-tétrahydronaphtalène, le complément à 100 % étant constitué par des quantités variables de décahydronaphtalène et de naphtalène.

Les exemples qui suivent illustrent l'invention.

### EXEMPLE 1 :

### Préparation d'une composition comprenant les composés (y), (y1) et (Y2)

Dans un réacteur de 10 I muni d'une agitation rotative, d'un réfrigérant ascendant, d'un injecteur d'azote, d'un gaine thermométrique, d'une ampoule de coulage et de moyens de chauffage, on charge 5 404 g de 1,2,3,4-tétrahydronaphtalène à 98,5 % de pureté soit 40,94 moles. Le produit est porté à 120°C sous balayage d'azote et agitation.

La sortie du réfrigérant est ensuite reliée à un barboteur à eau.

On ajoute dans le réacteur 6,7 g de FeCl₃ anhydre puis ensuite, toujours sous balayage d'azote, 1295,4 g de chlorure de benzyle à 99 % de pureté soit 10,24 moles en 3h30. Le rapport molaire 1,2,3,4-tétrahydronaphtalène / chlorure de benzyle est égal à 4.

En fin d'introduction du chlorure de benzyle, on note que la quantité d'HCI dégagé et piégé dans le barboteur est de 9,14 moles.

La teneur pondérale en chlorure de benzyle présente dans le milieu réactionnel est de 0,74 %.

La réaction est poursuivie sous agitation et balayage d'azote, pendant 1 heure à 120°C puis 1 heures à 130°C.

La quantité totale d'HCI dégagé et piégé dans le barboteur est égale à 10,2 moles.

La teneur finale en chlorure de benzyle dans le milieu réactionnel est d'environ 0,02 % en poids. Après refroidissement à température ambiante et sous balayage d'azote, le contenu du réacteur (6 325 g) est ensuite placé dans un ballon de distillation de 10 I surmonté d'une colonne adiabatique de 50 cm de haut, remplie par des ressorts en verre (efficacité de la colonne égale à environ 3 plateaux théoriques), surmontée d'une tête simple de distillation et d'un réfrigérant.

Le 1,2,3,4-tétrahydronaphtalène non transformé est récupéré par distillation sous une pression de 5,3 kPa (40 mm de mercure).

La distillation est réalisée en 4 heures à une température de pied allant de 130°C à 239°C et une température de tête allant de 115°C à 118°C pendant la majorité de la distillation avec, en fin de distillation, une montée à 142°C.

On récupère 4 200 g d'un liquide incolore ayant une teneur en 1,2,3,4-tetrahydronaphtalène supérieure à 98,5 % et peut être recyclé dans une opération ultérieure.

Le pied de distillation (2 105 g), contenant moins de 0,14 % en poids de 1,2,3,4-tétrahydronaphtalène est soumis ensuite à une opération d'élimination des faibles quantités de chlore organique résiduel, opération qui consiste à traiter ledit pied de distillation avec environ 21 g de CH₃ONa en poudre (1 % en poids par rapport au poids de produit à traiter) dans un réacteur sous agitation et, sous balayage d'azote, à 300°C pendant 3 heures.

Le produit ainsi traité, comprenant 77,3 % en poids de composés de formule (Y), 18,6 % en poids de composés de formule (Y1) et 2,8 % en poids de composés de formule (Y2), est soumis à une distillation fractionnée dans l'appareillage de distillation utilisé précédemment.

La distillation est conduite dans un premier temps sous une pression de 2,4 kPa (18 mm de Hg) à des températures de pied allant de 220°C à 294°C, puis dans un second temps, après élimination du garnissage de la colonne, sous une pression de 1,6 kPa (12 mm de Hg) à des températures de pied allant de 294°C à 344°C.

On récupère différentes fractions qui ont les teneurs pondérales (%) en mono-, et polybenzyl-1,2,3,4-tétrahydronaphtalènes telles que reportées dans le tableau 1 ci-après :

**TABLEAU 1**

| **Fraction** | **Poids (g)** | **Température d'ébullition (°C)** | **Pression kPa (mmHg)** | **BTHN (%)** | **DBTHN (%)** | **TBTHN (%)** |
|---|---|---|---|---|---|---|
| 1 | 124,5 | 200 - 218 | 2,4 (18) | 95,5 | - | - |
| 2 | 1 400 | 218 - 220 | 2,4 (18) | >98,8 | 0,2 | - |
| 3 | 13,5 | 220 - 290 | 1,6 (12) | 20,37 | 66,3 | 1,3 |
| 4 | 440 | 290 - 305 | 1,6 (12) | 0,44 | 92,2 | 5,2 |
| 5 | 69 | 305 - 330 | 1,6 (12) | 0,35 | 45,7 | 53,5 |
| 6 | 14 | 330 - 344 | 1,6 (12) | 0,7 | 4 | 93,3 |

Dans ce tableau 1 :
BTHN désigne le monobenzyl-1,2,3,4-tetrahydronaphtalène (formule (Y)) qui se trouve être un mélange de 6-benzyl-1,2,3,4-tetrahydronaphtalène (65 % en poids) et de 5-benzyl-1,2,3,4-tetrahydronaphtalène (35 % en poids).
DBTHN désigne les composés de formule (Y1).
TBTHN désigne un mélange de composés de formule (Y2) dans laquelle y+z+y'+y"+z'+z" =3.

Le résidu d'environ 40 g contient environ 20 % en poids de TBTHN ainsi que des produits lourds non identifiés et la partie minérale.

Les premières fractions sont des liquides incolores. Les dernières fractions sont jaunes.

Les compositions selon l'invention peuvent être aisément préparées (obtenues) en mélangeant les fractions précédemment obtenues par distillation de façon à obtenir une composition contenant en poids :
a % de BTHN (Y),
b % de DBTHN (Y1),
c % de TBTHN (Y2).

Ainsi, il a été obtenu une composition désignée ci-après PBTHN comprenant environ :
- 80 % en poids (79,96 %) d'un mélange d'isomères du monobenzyl-1,2,3,4-tetrahydronaphtalène (composés de formule (Y)) ;
- 19 % en poids (18,98 %) de composés de formule (Y1) ;
- 1 % en poids (1,06 %) de composés de formule (Y2) dans laquelle y+z+y'+y"+z'+z"=3)
en mélangeant par exemple 83 parties en poids de la fraction 2 avec 21 parties en poids de la fraction 4.

### EXEMPLE 2 :

### Préparation d'un mélange d'isomères du monobenzyl-1,2,3,4-tétrahydronaphtalène (Y)

Dans un réacteur de 6 litres muni d'une agitation rotative, d'un réfrigérant ascendant, d'un injecteur d'azote, d'un gaine thermométrique, d'une ampoule de coulage et de moyens de chauffage, on charge 3 890 g de 1,2,3,4-tétrahydronaphtalène à 98,5 % de pureté soit 22,97 moles. Le produit est porté à 120°C sous balayage d'azote et agitation.

La sortie du réfrigérant est ensuite reliée à un barboteur à eau.

On ajoute dans le réacteur 4,3 g de FeCl₃ anhydre puis ensuite, toujours sous balayage d'azote, 375 g de chlorure de benzyle à 99 % de pureté soit 2,95 moles en 3h30. Le rapport molaire 1,2,3,4-tétrahydronaphtalène / chlorure de benzyle est égal à 10.

En fin d'introduction du chlorure de benzyle, on note que la quantité d'HCI dégagé et piégé dans le barboteur est de 2,21 moles.

La réaction est poursuivie sous agitation et balayage d'azote, pendant 1 heure à 120°C.

La quantité totale d'HCI dégagé et piégé dans le barboteur est égale à 2,4 moles, une partie restant solubilisée dans le milieu réactionnel.

La teneur finale en chlorure de benzyle dans le milieu réactionnel est d'environ 0,02 % en poids. Après refroidissement à température ambiante et sous balayage d'azote, le contenu du réacteur (4144 g) est ensuite placé dans un ballon de distillation de 6 I surmonté d'une colonne adiabatique de 50 cm de haut, remplie par des ressorts en verre (efficacité de la colonne égale à environ 3 plateaux théoriques), surmontée d'une tête simple de distillation et d'un réfrigérant.

Le 1,2,3,4-tétrahydronaphtalène non transformé est récupéré par distillation sous une pression de 2,67 kPa (20 mm de mercure).

La distillation est réalisée en 4 heures à une température de pied allant de 105°C à 213°C et une température de tête allant de 92°C à 94°C pendant la majorité de la distillation avec, en fin de distillation, une montée à 159°C.

On récupère 3 483 g d'un liquide incolore ayant une teneur en 1,2,3,4-tetrahydronaphtalène supérieure à 98,5 % et peut être recyclé dans une opération ultérieure.

Le pied de distillation (647 g), contenant moins de 0,02 % en poids de 1,2,3,4-tétrahydronaphtalène est soumis ensuite à une opération d'élimination des faibles quantités de chlore organique résiduel, opération qui consiste à traiter ledit pied de distillation avec environ 6,5 g de CH₃ONa en poudre (1 % en poids par rapport au poids de produit à traiter) dans un réacteur sous agitation et, sous balayage d'azote, à 300°C pendant 3 heures.

Le produit ainsi traité, est soumis à une distillation fractionnée dans l'appareillage de distillation utilisé précédemment.

La distillation est conduite sous une pression de 2,4 kPa (18 mm de Hg) à des températures de pied allant de 220°C à 303°C.

On récupère 569 g d'un mélange d'isomères du monobenzyl-1,2,3,4-tetrahydronaphtalène qui se trouve être un mélange de 6-benzyl-1,2,3,4-tetrahydronaphtalène et de 5-benzyl-1,2,3,4-tetrahydronaphtalène ayant un point d'ébullition à pression atmosphérique de 350°C, une viscosité à 20°C égale à 21 mm²/s et une teneur en chlore égale à 1 ppm. Le rendement par rapport au chlorure de benzyle engagé est de 86,87 %.

Le mélange de 6-benzyl-1,2,3,4-tétrahydronaphtalène et de 5-benzyl-1,2,3,4-tetrahydronaphtalène a été caractérisé, d'une part par RMN du ¹³C dans CDCl₃ comme solvant (couplé et découplé ¹H) qui permet l'identification des structures des 2 isomères et RMN ¹H dans CDCl₃ et C₆D₆ qui permet de donner les proportions des 2 isomères, d'autre part par couplage chromatographie en phase gazeuse - spectrométrie de masse qui permet de confirmer la structure et également les proportions des 2 isomères. Notamment, le 5-benzyl-1,2,3,4-tétrahydronaptalène est caractérisé par un pic intense de fragmentation m/z égal à 144 qui est attribué au cation-radical obtenu par suite de l'élimination de benzène du cation radical 5-benzyl-1,2,3,4-tetrahydronaphtalène.

Les techniques utilisées indiquent que le mélange d'isomères du monobenzyl-1,2,3,4-tétrahydronaphtalène comprend 35 % en poids de 5-benzyl-1,2,3,4-tétrahydronaphtalène et 65 % en poids de 6-benzyl-1,2,3,4-tétrahydronaphtalène.

On a évalué la stabilité thermique de la composition préparée dans l'exemple 1, désignée par PBTHN ainsi que du mélange d'isomères du monobenzyl-1,2,3,4-tétrahydronaphtalène préparé dans l'exemple 2 et que l'on désigne par BTHN comparativement à un fluide de transfert de chaleur commercialisé par Dow CHEMICAL COMPANY sous la dénomination DOWTHERM RP qui est un mélange de 1,2,3,4-tetrahydro-5-(1phényléthyl)naphtalène et de 1,2,3,4-tetrahydro-6-(1-phényléthyl)-naphtalène.

Les essais ont été réalisés à 370°C pendant 160 heures pour le PBTHN et à 370°C pendant 160 heures et 340 heures pour le BTHN dans un autoclave de 200 ml en inox muni d'une gaine thermométrique et d'un manomètre. On charge 50 g de fluide de transfert de chaleur à tester, on effectue un balayage d'azote puis on ferme l'autoclave qui est ensuite placé dans un bain de sable chauffé électriquement. Une régulation permet de maintenir de façon constante la température des fluides de transfert de chaleur à 370°C.

Les résultats sont reportés dans les tableaux 2 et 3, et mettent en évidence le meilleur comportement thermique de la composition PBTHN et du BTHN selon l'invention.

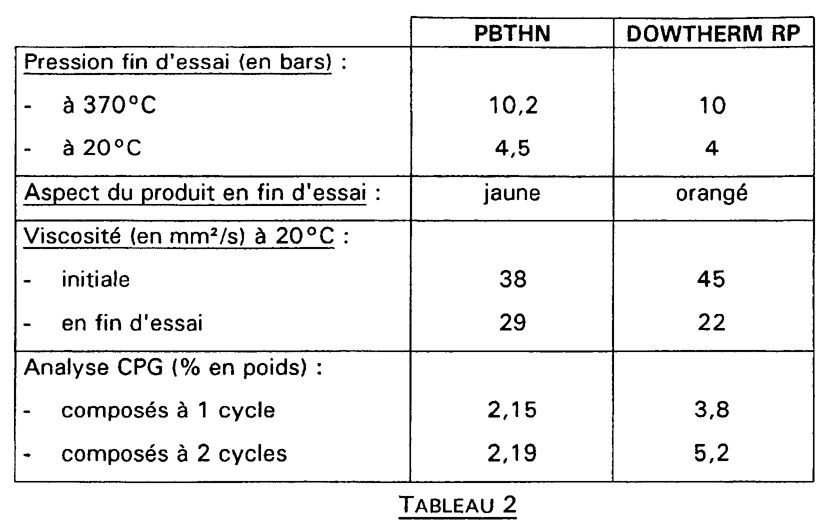

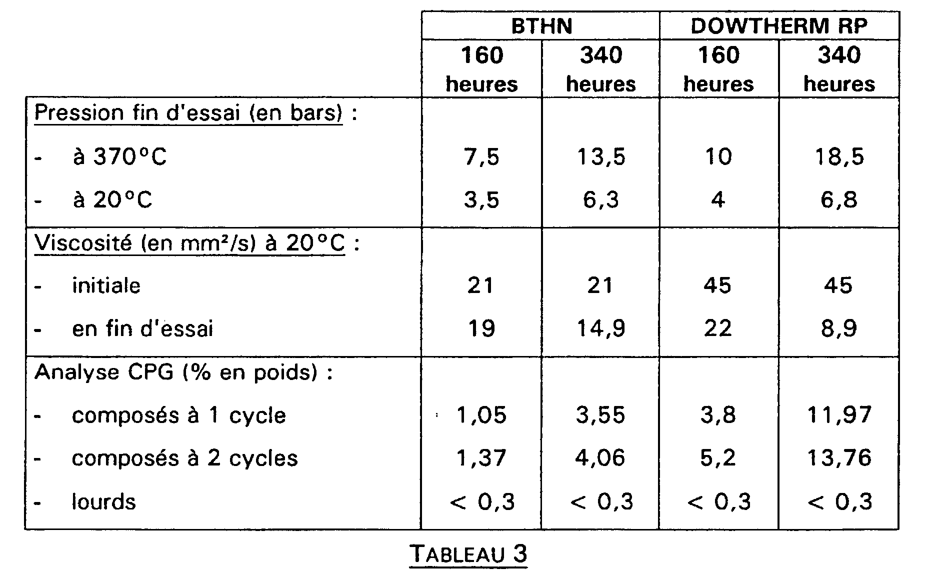

Dans ces tableaux, on entend par composés à 1 cycle des composés aromatiques tels que toluène, xylènes, éthylbenzène par composés à 2 cycles des composés aromatiques tels que naphtalène, méthylnaphtalène, 1,2,3,4-tétradydronaphtalène, méthyltétraline et par lourds des composés à plus de 3 cycles.

## Revendications

1. Compositions de mono- et de polybenzyl-1,2,3,4-tétrahydronaphtalène, **caractérisées en ce qu'**elles comprennent un mélange de composés (Y), (Y1) et (Y2) suivants :
◆ (Y) est un mélange d'isomères du monobenzyl-1,2,3,4-tétrahydronaphtalène de formule : 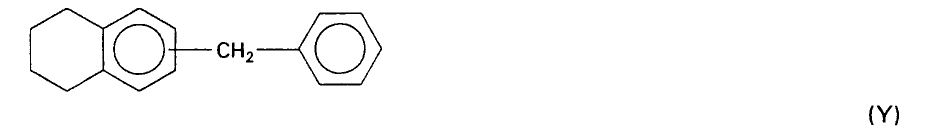
◆ (Y1) est un mélange de composés de monobenzylation de (Y) de formules : 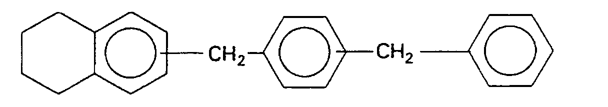 et 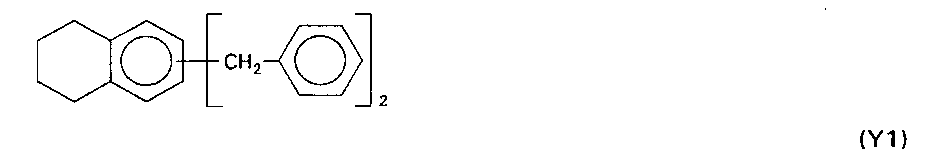
◆ (Y2) est un mélange de composés de mono- ou polybenzylation de (Y1) de formules : 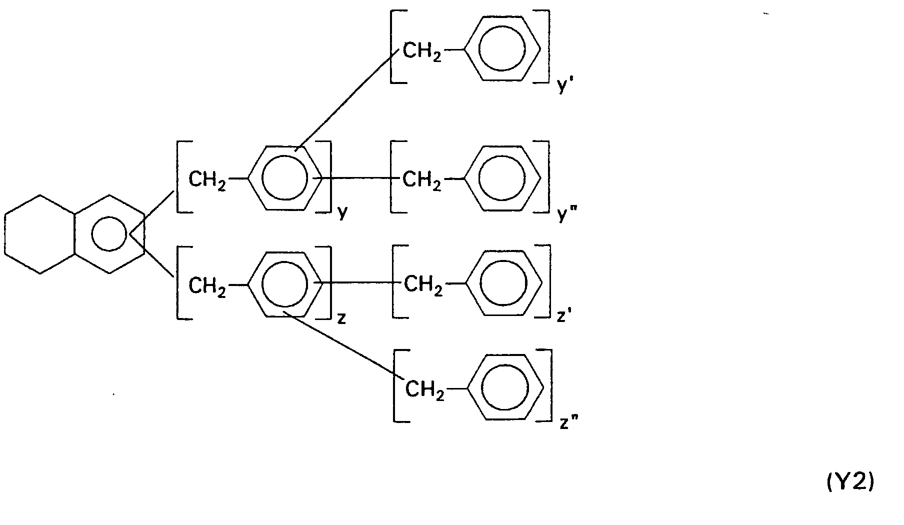 avec y et z=0, 1 ou 2 ; y', y", z', z"=0 ou 1 sachant que y+z n'est jamais égal à 0, que y'+y"+z'+z"≥1 et que y+z+y'+y"+z'+z" ≥3.

2. Compositions selon la revendication 1, **caractérisées en ce que** les composés (Y), (Y1) et (Y2) se trouvent dans les proportions pondérales suivantes :
- composés de formule (Y) compris entre 60 % et 90 %,
- composés de formule (Y1) compris entre 9 % et 35 %,
- composés de formule (Y2) compris entre 0,1 % et 10 %.

3. Composition selon la revendication 2, **caractérisée en ce qu'**elle comprend :
- 80 % en poids de composés de formule (Y),
- 19 % en poids de composés de formule (Y1),
- 1 % en poids de composés de formule (Y2).

4. Utilisation des compositions selon l'une des revendications 1 à 3 ou bien d'un mélange d'isomères du monobenzyl-1,2,3,4-tétrahydronaphtalène de formule : 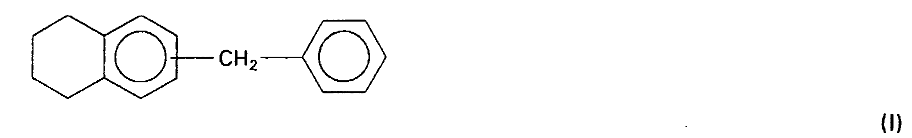 comme fluide de transfert de chaleur.

5. Utilisation selon la revendication 4, **caractérisée en ce que** le mélange d'isomères du monobenzyl-1,2,3,4-tétrahydronaphtalène comprend 30 % à 40 % en poids de 5-benzyl-1,2,3,4-tétrahydronaphtalène et 70 % à 60 % en poids de 6-benzyl-1,2,3,4-tétrahydronaphtalène.

6. Utilisation selon la revendication 5, **caractérisée en ce que** le mélange d'isomères du monobenzyl-1,2,3,4-tetrahydronaphtalène comprend 35 % en poids de 5-benzyl-1,2,3,4-tetrahydronaphtalène et 65 % en poids de 6-benzyl-1,2,3,4-tetrahydronaphtalène.

7. Procédé de fabrication des compositions de mono- et de polybenzyl-1,2,3,4-tétrahydronaphtalènes des revendications 1 à 3 ou bien du mélange d'isomères du monobenzyl-1,2,3,4-tétrahydronaphtalène des revendications 4 à 6, **caractérisé en ce que** l'on fait réagir du chlorure de benzyl sur du 1,2,3,4-tétrahydronaphtalène selon un rapport molaire 1,2,3,4-tétrahydronaphtalène / chlorure de benzyl de préférence égal à environ 4 lorsque l'on prépare les compositions comprenant un mélange de composés (Y), (Y1) et (Y2) et, au moins égal à 4, et, de préférence compris entre 8 et 12, lorsque l'on prépare le mélange d'isomères du monobenzyl-1,2,3,4-tétrahydronaphtalène, en présence d'un halogénure minéral ou d'un acide protonique à une température comprise entre 30°C et 150°C.

8. Procédé selon la revendication 7, **caractérisé en ce que**, pour préparer un mélange d'isomères du monobenzyl-1,2,3,4-tétrahydronaphtalène, l'on utilise un rapport molaire 1,2,3,4-tétrahydronaphtalène / chlorure de benzyl égal à 10.

9. Procédé selon l'une des revendications 7 ou 8, **caractérisé en ce que** la réaction terminée, on élimine l'excès de 1,2,3,4-tétrahydronaphtalène, on élimine l'halogénure minérale ou l'acide protonique puis on effectue sur le produit obtenu une distillation fractionnée.

10. Procédé selon la revendication 7, **caractérisé en ce que** l'halogénure minérale est le chlorure ferrique.

## Patentansprüche

1. Zusammensetzungen von Mono- und Polybenzyl-1,2,3,4-tetrahydronaphthalin, **dadurch gekennzeichnet, dass** sie eine Mischung von folgenden Verbindungen (Y), (Y1) und (Y2) umfassen:
• (Y) ist eine Isomerenmischung des Monobenzyl-1,2,3,4-tetrahydrohaphthalens mit folgender Formel: 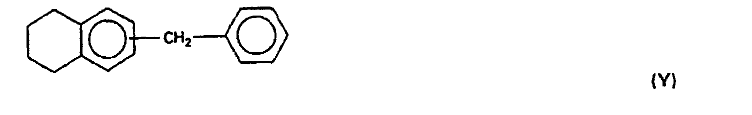
• (Y1) ist eine Mischung von Monobenzylationsverbindungen von (Y) mit den folgenden Formeln: 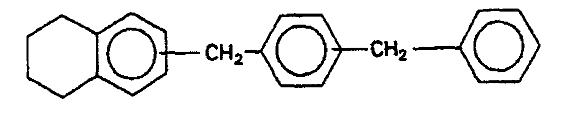 und 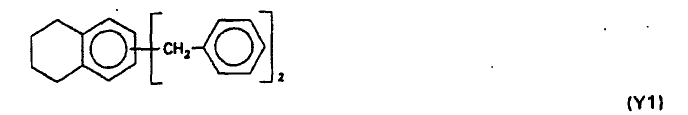
• (Y2) ist eine Mischung von Mono- oder Polybenzylationsverbindungen von (Y1) mit folgenden Formeln: 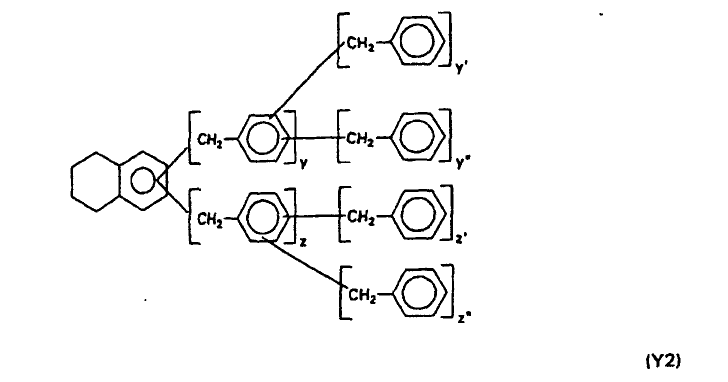
wobei y und z = 0, 1 oder 2; y', y'', z', z'' = 0 oder 1, wobei bekannt ist, dass y+z niemals gleich 0 ist, dass y'+y''+z'+z''≥1 und dass y+z+y'+y''+z'+z''≥3.

2. Zusammensetzungen nach Anspruch 1, **dadurch gekennzeichnet, dass** die Verbindungen (Y), (Y1) und (Y2) in folgenden Gewichtsverhältnissen vorhanden sind:
- Verbindungen mit der Formel (Y) zwischen 60 % und 90 %,
- Verbindungen mit der Formel (Y1) zwischen 9 % und 35 %,
- Verbindungen mit der Formel (Y2) zwischen 0,1 % und 10 %.

3. Zusammensetzung nach Anspruch 2, **dadurch gekennzeichnet, dass** sie umfasst:
- 80 Gew.-% von Verbindungen mit der Formel (Y),
- 19 Gew.-% von Verbindungen mit der Formel (Y1),
- 1 Gew.-% von Verbindungen mit der Formel (Y2).

4. Verwendung der Zusammensetzungen nach einem der Ansprüche 1 bis 3 oder auch einer Isomerenmischung des Monobenzyl-1,2,3,4-tetrahydronaphthalins mit folgender Formel: 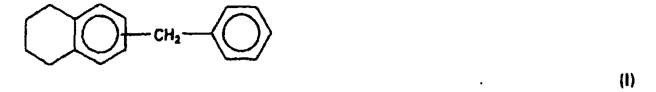 als Wärmeübertragungsfluid.

5. Verwendung nach Anspruch 4, **dadurch gekennzeichnet, dass** die Isomerenmischung des Monobenzyl-1,2,3,4-tetrahydronaphthalins 30 Gew.-% bis 40 Gew.-% von 5-Benzyl-1,2,3,4-tetrahydronaphthalin und 70 Gew.-% bis 60 Gew.-% von 6-Benzyl-1,2,3,4-tetrahydronaphthalin umfasst.

6. Verwendung nach Anspruch 5, **dadurch gekennzeichnet, dass** die Isomerenmischung des Monobenzyl-1,2,3,4-tetrahydronaphthalins 35 Gew.-% von 5-Benzyl-1,2,3,4-tetrahydronaphthalin und 65 Gew.-% von 6-Benzyl-1,2,3,4-tetrahydronaphthalin umfasst.

7. Verfahren zur Herstellung der Zusammensetzungen von Mono- und Polybenzyl-1,2,3,4-tetrahydronaphthalinen der Ansprüche 1 bis 3 oder auch der Isomerenmischung des Monobenzyl-1,2,3,4-tetrahydronaphthalins der Ansprüche 4 bis 6, **dadurch gekennzeichnet, dass** Benzylchlorid mit 1,2,3,4-Tetrahydronaphthalin in einem Molverhältnis 1,2,3,4-Tetrahydronaphthalin/Benzylchlorid von vorzugsweise gleich ungefähr 4, wenn die Zusammensetzungen hergestellt werden, die eine Mischung von Verbindungen (Y), (Y1) und (Y2) umfassen, und von mindestens gleich 4 und vorzugsweise zwischen 8 und 12, wenn die Isomerenmischung des Monobenzyl-1,2,3,4-tetrahydronaphthalins in Gegenwart eines mineralischen Halogenids oder einer Protonsäure bei einer Temperatur zwischen 30 °C und 150 °C hergestellt wird, zur Reaktion gebracht wird.

8. Verfahren nach Anspruch 7, **dadurch gekennzeichnet, dass** für die Herstellung einer Isomerenmischung des Monobenzyl-1,2,3,4-tetrahydronaphthalins ein Molverhältnis 1,2,3,4-Tetrahydronaphthalin/Benzylchlorid gleich 10 verwendet wird.

9. Verfahren nach einem der Ansprüche 7 oder 8, **dadurch gekennzeichnet, dass**, wenn die Reaktion beendet ist, der Überschuss von 1,2,3,4-Tetrahydrohaphthalen beseitigt wird, das mineralische Halogenid oder die Protonsäure beseitigt wird und dann auf dem erhaltenen Produkt eine fraktionierte Destillation durchgeführt wird.

10. Verfahren nach Anspruch 7, **dadurch gekennzeichnet, dass** das mineralische Halogenid Eisenchlorid ist.

## Claims

1. Mono- and polybenzyl-1,2,3,4-tetrahydronaphthalene compositions, **characterized in that** they comprise a mixture of the following compounds (Y), (Y1), and (Y2):
◆ (Y) is a mixture of monobenzyl-1,2,3,4-tetrahydronaphthalene isomers of formula: 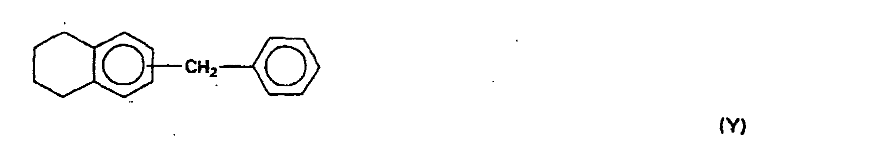
◆ (Y1) is a mixture of monobenzylation compounds of (Y), of formulae: 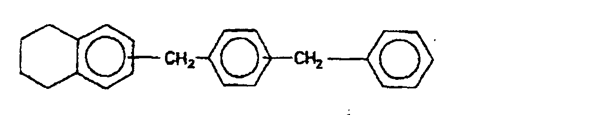 and 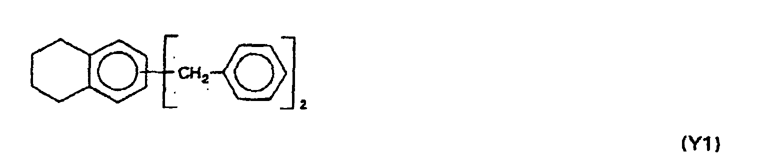
◆ (Y2) is a mixture of mono- or polybenzylation compounds of (Y1), of formulae: 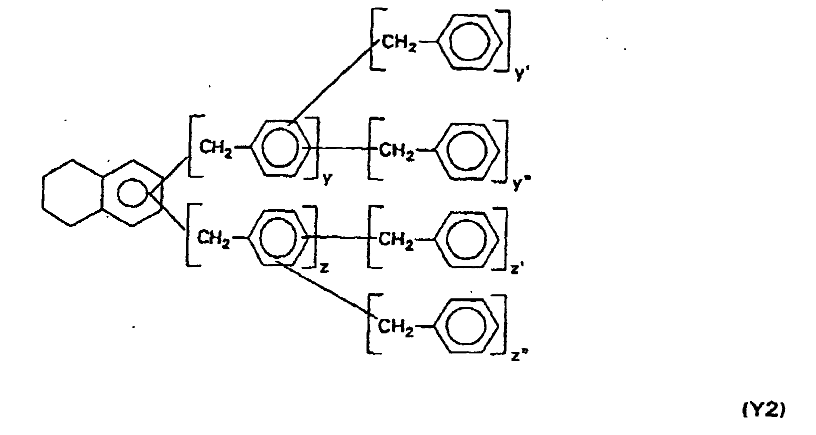
where y and z = 0, 1 or 2; y', y", z', z" = 0 or 1, with the provisos that y + z is never 0, that y' + y" + z' + z" ≥ 1, and that y + z + y' + y" + z' + z" ≥ 3.

2. Compositions according to Claim 1, **characterized in that** the compounds (Y), (Y1), and (Y2) are present in the following proportions by weight:
- compounds of formula (Y): between 60% and 90%,
- compounds of formula (Y1): between 9% and 35%,
- compounds of formula (Y2): between 0.1% and 10%.

3. Composition according to Claim 2, **characterized in that** it comprises:
- 80% by weight of compounds of formula (Y);
- 19% by weight of compounds of formula (Y1);
- 1% by weight of compounds of formula (Y2).

4. Use of compositions according to one of Claims 1 to 3 or else of a mixture of monobenzyl-1,2,3,4-tetrahydronaphthalene isomers of formula: 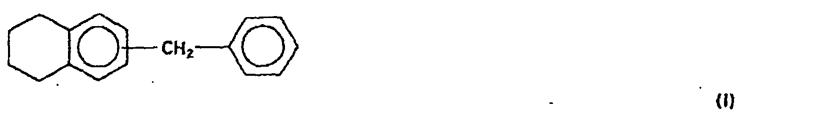 as heat transfer fluid.

5. Use according to Claim 4, **characterized in that** the mixture of monobenzyl-1,2,3,4-tetrahydronaphthalene isomers comprises from 30% to 40% by weight of 5-benzyl-1,2,3,4-tetrahydronaphthalene and from 70% to 60% by weight of 6-benzyl-1,2,3,4-tetrahydronaphthalene.

6. Use according to Claim 5, **characterized in that** the mixture of monobenzyl-1,2,3,4-tetrahydronaphthalene isomers comprises 35% by weight of 5-benzyl-l,2,3,4-tetrahydronaphthalene and 65% by weight of 6-benzyl-1,2,3,4-tetrahydronaphthalene.

7. Process for preparing mono- and polybenzyl-1,2,3,4-tetrahydronaphthalene compositions of Claims 1 to 3 or else the mixture of monobenzyl-1,2,3,4-tetrahydronaphthalene isomers of Claims 4 to 6, **characterized in that** benzyl chloride is reacted with 1,2,3,4-tetrahydronaphthalene in a molar 1,2,3,4-tetrahydronaphthalene/benzene chloride ratio of preferably equal to approximately 4 when preparing compositions comprising a mixture of compounds (Y), (Y1), and (Y2), and at least equal to 4 and, preferably, between 8 and 12 when preparing the monobenzyl-1,2,3,4-tetrahydronaphthalene isomer mixture in the presence of an inorganic halide or a protic acid at a temperature of between 30°C and 150°C.

8. Process according to Claim 7, **characterized in that** for preparing a mixture of monobenzyl-1,2,3,4-tetrahydronaphthalene isomers a molar 1,2,3,4-tetrahydronaphthalene/benzyl chloride ratio of equal to 10 is used.

9. Process according to either of Claims 7 and 8, **characterized in that**, when the reaction is at an end, the excess 1,2,3,4-tetrahydronaphthalene is removed, the inorganic halide or the protic acid is removed, and then the product obtained is subjected to fractional distillation.

10. Process according to Claim 7, **characterized in that** the inorganic halide is ferric chloride
